# EUROPEAN PATENT SPECIFICATION

(11) **EP 3 156 408 B1**
(45) Date of publication and mention of the grant of the patent: **12.12.2018**
(21) Application number: 15867780.7
(22) Date of filing: 19.10.2015
(51) Int. Cl.: C07F 7/24, H01L 31/042, H01L 51/00, H01L 51/42

(54) **ORGANIC-INORGANIC HYBRID PEROVSKITE COMPOUND, METHOD FOR PREPARING SAME, AND SOLAR CELL COMPRISING SAME**
HYBRIDE ORGANISCH-ANORGANISCHE PEROVSKITEVERBINDUNG, VERFAHREN ZUR HERSTELLUNG DAVON UND SOLARZELLE DAMIT
COMPOSÉ DE PÉROVSKITE HYBRIDE ORGANIQUE-INORGANIQUE, PROCÉDÉ POUR SA PRÉPARATION, ET CELLULE SOLAIRE LE COMPRENANT

(30) Priority: 08.12.2014 KR 20140174979; 15.10.2015 KR 20150144202
(43) Date of publication of application: 19.04.2017
(73) Proprietor: LG Chem, Ltd., Seoul 07336 (KR)
(72) Inventor: PARK, Eun Seok, Daejeon 34122 (KR); HONG, Sung Kil, Daejeon 34122 (KR); CHUN, Sung-Ho, Daejeon 34122 (KR); YOO, Dong Woo, Daejeon 34122 (KR)
(74) Representative: Hoffmann Eitle
(86) International application number: PCT/KR2015/011034
(87) International publication number: WO 2016/093485

(56) References cited:
- WO-A1-2013/171517
- KR-A- 20140 007 045
- KR-B1- 101 462 025
- KR-B1- 101 561 284
- ONODA-YAMAMURO ET AL: "Neutron-diffraction study of CD"3ND"3SnBr"3: Semiconductor-insulator transition with orientational ordering", PHYSICA B: CONDENSED MA, ELSEVIER, AMSTERDAM, NL, vol. 213-214, 1 August 1995 (1995-08-01), pages 411-413, XP005257609, ISSN: 0921-4526, DOI: 10.1016/0921-4526(95)00173-7
- STEIJGER J J M ET AL: "Structural and magnetic phase transitions in the layered compound (CD"3ND"3)"2CuCl"4", JOURNAL OF MAGNETISM AND MAGNETIC MATERIALS, ELSEVIER, AMSTERDAM, NL, vol. 14, no. 2-3, 2 December 1979 (1979-12-02), pages 152-154, XP024443466, ISSN: 0304-8853, DOI: 10.1016/0304-8853(79)90104-5 [retrieved on 1979-12-02]
- KOJI YAMADA ET AL: "Static and dynamic structures of CD3ND3GeCl3 studied by TOF high resolution neutron powder diffraction and solid state NMR", JOURNAL OF THE CHEMICAL SOCIETY, DALTON TRANSACTIONS., no. 10, 13 May 2002 (2002-05-13), pages 2112-2118, XP055409361, GB ISSN: 1472-7773, DOI: 10.1039/b201611g
- AKIHIRO KOJIMA ET AL: "Organometal Halide Perovskites as Visible-Light Sensitizers for Photovoltaic Cells", JOURNAL OF THE AMERICAN CHEMICAL SOCIETY, vol. 131, no. 17, 6 May 2009 (2009-05-06), pages 6050-6051, XP55045648, ISSN: 0002-7863, DOI: 10.1021/ja809598r
- KIM, HAK - BEOM ET AL.: 'Mixed Solvents for the Optimization of Mrphology in Solution-Processed, Inverted-Type Perovskite/Fullerene Hybrid Solar Cells' NANOSCALE vol. 6, 04 April 2014, pages 6679 - 6683, XP055392621

## Description

### [TECHNICAL FIELD]

The present invention relates to a solar cell comprising an organic-inorganic hybrid perovskite compound having a deuterium, and its preparation method.

### [BACKGROUND ART]

In order to solve the depletion of fossil energy and global environmental problems caused by the use thereof, studies of renewable and pure alternative energy sources such as solar energy, wind power and water power have been actively promoted.

Of these, interest in solar cells that can convert energy from sunlight directly into electrical energy has increased significantly. Here, the solar cell refers to a cell for generating current-voltage by utilizing the photovoltaic effect which absorbs light energy from sunlight to generate electrons and holes.

Currently, the n-p type diode silicon (Si) single crystal-based solar cells having a light energy conversion efficiency of 20% or more can be prepared and thus used for actual photovoltaic power generation. Solar cells using compound semiconductors such as gallium arsenide (GaAs) having superior conversion efficiencies than the above diode Si single crystal-based solar cells can also be used. However, since these inorganic semiconductor-based solar cells require materials purified at a high purity for high efficiency, more energy is consumed for the purification of the raw materials. Also, in the process of forming a single crystal or a thin film using the raw materals, expensive process equipments are required and thus there is a limitation in reducing production costs, which has become an obstacle to the large-scale utilization.

Accordingly, in order to produce solar cells at low cost, it is necessary to greatly reduce the cost of the core materials of the solar cells and the producting process thereof. As an alternative for the inorganic semiconductor-based solar cells, a dye-sensitized solar cell and an organic solar cell which can be manufactured by low-cost materials and processes have been actively studied.

The dye-sensitized solar cell (DSSC) was first developed by professor, Michael Gratzel, of Lausanne University (EPFL) in Switzerland in 1991 and introduced in Nature paper (Vol. 353, p. 737).

The initial dye-sensitized solar cell has a simple structure, filled with liquid electrolyte, in which the dye absorbing light in the porous photoanode is absorbed on the transparent electrode film flowing through light and electricity and then another conductive glass substrate is positioned on the upper part. The operating principle of the dye-sensitized solar cells is that, if the dye molecule chemically adsorbed on the porous photoanode surface absorbs sunlight, the dye molecule generates electronic-hole pairs; the electrons are injected in the conduction band of semiconductor oxides used as the porous photoanode and transferred to the transparent conductive film to generate current. The holes remaining in the dye molecule is transmitted to a photocathode by the hole conduction or hole-conducting polymer due to the oxidation-reduction reaction of a liquid or solid-state electrolyte and constitutes a complete solar cell circuit which makes it to work outside.

In the constitution of these dye-sensitized solar cells, the transparent conductive film is mainly composed of FTO(Fluorine doped Tin Oxide) or ITO(Indium doped Tin Oxide). For the porous photoanode, the nanoparticles with a broad band gap is used. For the dye, a variety of materials in which light is particularly well absorbed, the LUMO (lowest unoccupied molecular orbital) energy level of the dye is higher than the conduction band energy level of the photoanode material, and the separation of the excitons generated by light is easy, thereby increasing the efficiency of solar cells, are chemically synthesized and used. The maximum efficiency of the liquid dye-sensitized solar cell reported to date have remained 11-12% for about 20 years. The efficiency of the liquid dye-sensitized solar cell may be relatively high and is likely to be commonly used. However, there were problems in the stability with time due to the volatile liquid electrolyte and in reducing cost due to the use of costly ruthenium (Ru)-based dye.

To solve these problems, instead of the volatile liquid electrolyte, using a non-volatile electrolyte with ionic solvent, using a polymer gel type electrolyte and using low-cost pure organic dye have been studied. However, there was a problem in that efficiency is low as compared to the dye-sensitized solar cell using a volatile liquid electrolyte and ruthenium-based dyes.

On the other hand, organic photovoltaic (OPV) cells whose full-fledged research began from the mid-1990 consist of organic materials having both an electron donor (D, or sometimes referred to as a hole acceptor) property and an electron acceptor (A) property. When solar cells consisting of organic moleculars absorb the light, electrons and holes are formed, which are called exciton. The exciton moves to a D-A interface to separate a charge. The electron moves to an electron acceptor and the hole moves to an electron donor, thereby generating a photocurrent.

The distance that the excitons generated in the electron donor could be normally moved was around 10 nm which was very short. Therefore, the photoactive organic material could not be built thickly and thus light absorbance was low and efficiency was low. However, in recent years, along with the introduction of so-called "BHJ" (bulk heterojuction) concept which increases the surface area of the interface, and with the development of electron donor organic materials with a small band gap which is easy in a wide range of solar absorption, the efficiency was greatly increased. Thus, the organic solar cells having the efficiency of 8% or more has been reported (Advanced Materials, 23 (2011)4636).

Since organic solar cells have easy processability and versatility of organic materials and their cost is low, the manufacturing process of the device is simple as compared to existing solar cells. Therefore, low-cost production can be accomplished as compared with conventional solar cells. However, for the organic solar cells, the structure of BHJ is easily deteriorated by moisture or oxygen in air and thus the efficiency is rapidly decreased, that is, there is a big problem in the stability of the solar cells. In a method for solving this, the introduction of complete sealing technology can lead to an increase in the stability, but there is a problem where the cost is increased.

In a method for solving the problems encountered with the liquid dye-sensitized solar cells with the liquid electrolyte, Michael Gratzel, a professor of Chemical Department of Lausanne University in Switzerland and the inventor of the dye-sensitized solar cells, has reported solid dye-sensitized solar cells with the efficiency of 0.74% using Spiro-OMeTAD (2,2',7,7'-tetrakis (N,N-di-p-methoxyphenylamine)-9,9'-spirobifluorine), a solid hole conductive organic material, instead of a liquid electrolyte, in the Nature paper in 1998. Subsequently, the efficiency was increased up to about 6% by optimizing the structure and improving the interface properties and the hole conductivity. In addition, the solar cells using a low-cost, pure organic material as a ruthenium-based dye, and P3HT, PEDOT and the like as a hole conductor was produced, but the efficiency is 2-7% which is still low.

Also, studies of using the quantum dot nanoparticles as a light-absorbing material instead of the dye, and using the hole conductive inorganic or organic materials instead of the liquid electrolyte have been reported. The solar cells using CdSe, PbS and the like as the quantum dots and using a conductive polymer such as Spiro-OMeTAD or P3HT as a hole conductive polymer have been reported in numerous papers, but their efficiency is still 5% or less which is very low. Further, it has been reported that the solar cells using Sb2S3 as the light-absorbing inorganic material and using PCPDTBT as the hole-conducting organic materials have the efficiency of about 6% (Nano Letters, 11 (2011) 4789). However, more efficiency improvement has not been reported.

Further, it has been reported that the solar cells using a material having organic-inorganic hybrid perovskite structure rather than the quantum dot consisting of pure inorganic material, instead of the dye of the dye-sensitized solar cells, have the efficiency of about 9% (Scientific Reports 2, 591). Currently, it is rapidly developing to such an extent that the solar cells having about 20% are reported. In addition, although solar cells using a perovskite are published, reports for novel perovskite materials are deficient.

Akihiro Kojima et al in the Journal of the American Chemical Society vol. 131, no. 17, pages 6050 - 6051 disclose organometal halide perovskites as visible-light sensitizers for photovoltaic cells. According to this document, two organolead halide perovskite nanocrystals, CH₃NH₃PbBr₃ and CH₃NH₃PbI₃, were used to sensitize TiO₂ for visible-light conversion in photoelectrochemical cells.

Given the above circumstances, the present inventors have conducted extensive studies to change an organic-inorganic hybrid perovskite structure in order to enhance the stability of solar cells. As a result, the inventors have found that, when deuterium is substituted in the organic-inorganic hybrid perovskite structure comprised in the solar cell of the invention, the zero point energy becomes low and the perovskite compounds are chemically stabilized, thereby improving the stability of solar cells. The present invention was completed on this basis.

### [DISCLOSURE OF INVENTION]

### [TECHNICAL PROBLEM]

It is an object of the present invention to provide a solar cell comprising an organic-inorganic hybrid perovskite compound having a strucure in which deuterium is substituted in order to improve the stability of solar cells.

It is another object of the present invention to provide a process for preparing the above-described solar cell comprising the organic-inorganic hybrid perovskite compound.

### [TECHNICAL SOLUTION TO PROBLEM]

In order to achieve these objects, and other objects which will be become apparent from the description, the present invention provides a solar cell comprising an organic-inorganic hybrid perovskite compound, wherein the organic-inorganic hybrid perovskite compound is represented by the following chemical formula 1 or 2:

[Formula 1] AMX₃

[Formula 2] A₂MX₄

in the above formulas,
A is CD₃₋ₐHₐN⁺D_{3-b}H_{b}, wherein a is a real number from 0 to 3, b is a real number from 0 to 3, except i) when a is 3 and b is 3, ii) when a is 3 and b is 0, or iii) when a is 0 and b is 3,
M is a divalent metal ion, and
X is a halogen ion.

As used herein, the term "perovskite" is named after Russian mineralogist, Lev Perovski, and refers to any material with the same type of structure as calcium titanium oxide (CaTiO₃), the first perovskite type material, which was discovered in the Ural mountains. As shown in the above chemical formulas, the cations (A and M) and anion (X) consist of the chemcial formula: AMX₃ or A₂MX₄.

In the case of the perovskite used for the solar cells in the present invention, a common monovalent ammonium salt is used as a cation corresponding to A, whereby the term "organic-inorganic hybrid" is used.

As the perovskite used in a conventional solar cell, CH₃NH₃PbI₃ using methyl ammonium salt is representative. However, the above material has a high band gap and thus a limitation in increasing the efficiency of solar cells and there is a problem that it is chemically unstable. Therefore, according to the present invention, the perovskite compound in the solar cell is characterized by having a structure in which deuterium is substituted instead of hydrogen in order to improve the chemical stability of the perovskite compound.

The salt corresponding to A in the chemical formulas 1 and 2 is CD₃₋ₐHₐN⁺D_{3-b}H_{b}, wherein a is a real number from 0 to 3, b is a real number from 0 to 3, except when both a and b are 3, and thus A has at least one deuterium. In particular, there is a characteristic that at least one of the hydrogen which is substituted directly with the nitrogen is deuterium. Therefore, as compared with conventional perovskite having only hydrogen, the solar cell comprising the perovskite compound of the present invention has a lower zero point energy and thus it can be more chemically stabilized.

Preferably, in the chemical formulas 1 and 2, a and b are 0. In this case, hydrogen of the salt corresponding to A all has a structure (CD₃N⁺D₃) substituted by deuterium.

Further, in the chemical formulas 1 and 2, M is Pb²⁺, Sn²⁺, Ti²⁺, Nb²⁺, Zr²⁺ or Ce²⁺, and more preferably Pb²⁺.

Further, in the chemical formulas 1 and 2, X is Cl⁻, Br⁻ or I⁻.

According to one embodiment of the present invention, the solar cell comprising the organic-inorganic hybrid perovskite compound of the chemical formulas 1 and 2 in which deuterium is substituted according to the present invention has been identified to have improved stability as comprared with CD₃NH₃PbI₃ that was conventionally used. This results from subsitution of hydrogen with deuterium.

Further, the present invention provides a method for preparing the solar cell comprising the organic-inorganic hybrid perovskite compound represented by the below chemical formulas 1 and 2 comprising the following steps.
1) mixing a compound represented by the following chemical formula 3 with a compound represented by the following chemical formula 4, and
2) heat-treating the mixture.

   [Formula 3] AX

   [Formula 4] MX₂

   in the above formulas, A, M and X are the same as previously defined

In step 1 above, the molar ratio of the compound represented by the formula 3 and the compound represented by the formula 4 is preferably about 1:1.

As explained above, the present invention provides a solar cell which comprises the organic-inorganic hybrid perovskite compound represented by the chemical formulas 1 and 2.

The organic-inorganic hybrid perovskite compound of the chemical formulas 1 and 2 comprised in the solar cells according to the present invention serves to absorb solar light and thus it may constitute the light-absorbing layer in solar cells. Accordingly, the solar cell using the organic-inorganic hybrid perovskite compound according to the present invention may have the following structure:
a first electrode including a conductive transparent substrate;
a light-absorbing layer, formed on the first electrode, comprising the organic-inorganic hybrid perovskite compound represented by the chemical formulas 1 and 2;
a second electrode disposed opposite to the first electrode in which the light-absorbing layer is formed; and
an electrolyte layer located between the first electrode and the second electrode.

Further, the above-mentioned solar cell may be prepared from the following steps:
1) absorbing the organic-inorganic hybrid perovskite compound represented by the chemical formulas 1 and 2 on a first electrode comprising a conductive transparent substrate and then subjecting to heat treatment to form a light-absorbing layer;
2) disposing a second electrode opposite to the first electrode in which the light-absorbing layer is formed; and
3) injecting an electrolyte solution located between the first electrode and the second electrode to form an electrolyte layer.

The step 1 of absorbing the organic-inorganic hybrid perovskite compound may be conducted by a spin-coating, a dip coating, a screen coating, a spray coating, an electric spinning or the like for 10 seconds to 5 minutes. Further, the solvent for dispersing the organic-inorganic hybrid perovskite compound is not particularly limited as long as the solvent can easily dissolve the perovskite. However, gamma-butyrolactone, DMF and the like are preferable. After the absorption, the heat-treatment temperature is preferably 40 to 300°C.

The conductive transparent substrate can be doped with a material selected from the group consisting of Ti, In, Ga and Al.

For the second electrode, a conductive layer comprising at least one material selected from the group consisting of Pt, Au, Ni, Cu, Ag, In, Ru, Pd, Rh, Ir, Os, C and a conductive polymer, may be formed on a glass substrate or a plastic substrate including at least one material selected from the group consisting of ITO, FTO, ZnO-Ga₂O₃, ZnO-Al₂O₃ and tin oxide

The electrolyte solution that can be used includes those wherein an iodine and an additive are dissolved in a solvent. For example, at least one additive selected from the group consisting of urea, thiourea, tert-butylpyridine and guanidium thiotianate, and at least one solvent selected from the group consisting of ethylacetate, acetonitrile, toluene and methoxy propionitrile can be used, but they are not limited thereto.

### [ADVANTAGEOUS EFFECTS OF THE INVENTION]

For the organic-inorganic hybrid perovskite compounds comprised in the solar cells according to the present invention, since deuterium is substituted, a zero point energy becomes lower and the chemical stability of the perovskite compounds increases, thereby increasing the stability of the solar cells. Accordingly, the organic-inorganic hybrid perovskite compound comprised in the solar cells according to the present invention can make the best use of a light-absorbing material.

### [BRIEF DESCRIPTION OF THE DRAWING]

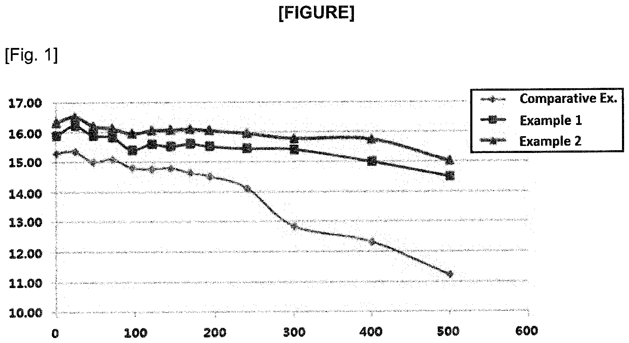

Fig. 1 shows the power conversion efficiency measured with the lapse of time for the solar cells prepared in one example of the present invention and a comparative example.

### [MODE FOR THE INVENTION]

Below, the preferred embodiments are presented to aid in the understanding of the invention.

### Preparation Example 1: Preparation of Methyl ammonium iodide-d₅

Methyl amine-d₅ gas was injected and filled into a 2L vacuum flask. The flask was maintained for about 15 minutes until gas had condensed at-78°C (dry ice and acetone bath) to make liquid. In the flask, 5 g of methanol-d₄ was injected by a syringe and stirred for 30 minutes. Then, the temperature was raised to 0°C. Hydriodic acid (HI 57 wt%) (22.66 mL, 0.17 mol) was injected and then stirred for 1.5 hours. The solvent was removed by the rotary evaporator, washed with diethyl ether, filtered and dried in vacuum to produce methyl ammonium iodide-d₅ (10.98 g, 80.4%).
²H NMR (spinning rate = 18 kHz) (600 MHz, ppm): 1.1 (3, s) 5.2 (2, s)
¹H NMR (D₂O (700 MHz), ppm): 4.28 (1, s)

### Preparation Example 2: Preparation of Methyl ammonium bromide-d₅

Methyl ammonium bromide-d₅ was prepared in the same manner as in Preparation Example 1, except that hydrobromic acid (HBr 48 wt%) was used instead of hydriodic acid (HI 57 wt%).
²H NMR (spinning rate = 18 kHz) (600 MHz, ppm): 1.2 (3, s) 5.3 (2, s)
¹H NMR (D₂O(700 MHz), ppm): 4.30 (1, s)

### Preparation Example 3: Preparation of Methyl ammonium chloride-d₅

Methyl ammonium chloride-d₅ was prepared in the same manner as in Preparation Example 1, except that hydrochloric acid (HBr 37 wt%) was used instead of hydriodic acid(HI 57 wt%).
²H NMR (spinning rate = 18 kHz) (600 MHz, ppm): 1.2 (3, s) 5.4 (2, s)
¹H NMR (D₂O(700 MHz), ppm): 4.31 (1, s)

### Preparation Example 4: Preparation of Methyl ammonium fluoride-d₅

Methyl ammonium fluoride-d₅ was prepared in the same manner as in Preparation Example 1, except that hydrofluoric acid (HF 48 wt%) was used instead of hydriodic acid (HI 57 wt%).
²H NMR (spinning rate = 18 kHz) (600 MHz, ppm): 1.2 (3, s) 5.5 (2, s)
¹H NMR (D₂O (700 MHz), ppm): 4.32 (1, s)

### Preparation Example 5: Preparation of Methyl ammonium iodide-d₆

Methyl ammonium iodide-d₆ was prepared in the same manner as in Preparation Example 1, except that deuterium iodide (DI 57 wt%) was used instead of hydriodic acid (HI 57 wt%).
²H NMR (spinning rate = 18 kHz) (600 MHz, ppm): 1.1 (3, s) 5.2 (3, s)

### Preparation Example 6: Preparation of Methyl ammonium bromide-d₆

Methyl ammonium bromide-d₆ was prepared in the same manner as in Preparation Example 2, except that deuterium bromide(DBr 47 wt%) was used instead of hydrobromic acid (HBr 48 wt%).
²H NMR (spinning rate = 18 kHz) (600 MHz, ppm): 1.2 (3, s) 5.3 (3, s)

### Preparation Example 7: Preparation of Methyl ammonium chloride-d₆

Methyl ammonium chloride-d₆ was prepared in the same manner as in Preparation Example 3, except that deuterium chloride(DCI 35 wt%) was used instead of hydrochloric acid (HCI 37 wt%).
²H NMR (spinning rate = 18 kHz) (600 MHz, ppm): 1.2 (3, s) 5.4 (3, s)

### Example 1: Preparation of Methyl ammonium lead iodide-d₅

Methyl ammonium iodide-d₅ prepared in Preparation Example 1 and lead iodide(II) were used at a mole ratio of 1:1, dissolved in 1-methyl-2-pyrrolidone and then stirred at a temperatrue of 70°C for 2 hours to produce 40 wt% of methyl ammonium lead iodide-d₅ solution.

### Example 2: Preparation of Methyl ammonium lead bromide-d₅

Methyl ammonium bromide-d₅ prepared in Preparation Example 2 and lead iodide(II) were used at a mole ratio of 1:1, dissolved in 1-methyl-2-pyrrolidone and then stirred at a temperatrue of 70°C for 2 hours to produce 40 wt% of methyl ammonium lead bromide-d₅ solution.

### Example 3: Preparation of Methyl ammonium lead chloride-d₅

Methyl ammonium chloride-d₅ prepared in Preparation Example 3 and lead iodide(II) were used at a mole ratio of 1:1, dissolved in 1-methyl-2-pyrrolidone and then stirred at a temperatrue of 70°C for 2 hours to produce 40 wt% of methyl ammonium lead chloride-d₅ solution.

### Example 4: Preparation of Methyl ammonium lead fluoride-d₅

Methyl ammonium fluoride-d₅ prepared in Preparation Example 4 and lead iodide(II) were used at a mole ratio of 1:1, dissolved in 1-methyl-2-pyrrolidone and then stirred at a temperatrue of 70°C for 2 hours to produce 40 wt% of methyl ammonium lead fluoride-d₅ solution.

### Example 5: Preparation of Methyl ammonium lead iodide-d₆

Methyl ammonium iodide-d₆ prepared in Preparation Example 5 and lead iodide(II) were used at a mole ratio of 1:1, dissolved in 1-methyl-2-pyrrolidone and then stirred at a temperatrue of 70°C for 2 hours to produce 40 wt% of methyl ammonium lead iodide-d₆ solution.

### Example 6: Preparation of Methyl ammonium lead bromide-d₆

Methyl ammonium bromide-d₆ prepared in Preparation Example 6 and lead bromide(II) were used at the mole ratio of 1:1, dissolved in 1-methyl-2-pyrrolidone and then stirred at a temperatrue of 70°C for 2 hours to produce 40 wt% of methyl ammonium lead bromide-d₆ solution.

### Example 7: Preparation of Methyl ammonium lead bromide-d₆

Methyl ammonium bromide-d₆ prepared in Preparation Example 5, methyl ammonium bromide-d₆ prepared in Preparation Example 6 lead iodide(II) and lead bromide(II) were used at a mole ratio of 9:1:9:1, dissolved in 1-methyl-2-pyrrolidone and then stirred at a temperatrue of 70°C for 2 hours to produce 40 wt% of methyl ammonium lead iodide bromide-d₆ solution.

### Comparative Example: Preparation of Methyl ammonium iodide

Methyl amine solution (40 wt% in methanol) (30 mL) was injected into a 250mL flask and cooled in ice bath at 0°C. Hydriodic acid (HI 57 wt%) (32.3 mL) was injected and then stirred for 1.5 hours. The solvent was removed by the rotary evaporator, washed with diethyl ether, filtered and dried in vacuum to produce methyl ammonium iodide(yield: 80.4%). The produced methyl ammonium iodide and and lead iodide(II) were used at a mole ratio of 1:1, dissolved in 1-methyl-2-pyrrolidone and then stirred at a temperatrue of 70°C for 2 hours to produce 40 wt% of methyl ammonium lead iodide solution.

### Experimental Example

FTO glass (Pikington, TEC-7, 7 Ω/sq) was washed in ethanol using ultrasound for 40 minutes. The FTO substrate was coated by a spin coating method using 0.1M titanium(IV) bis(ethyl acetoacetato)diisopropoxide/1-butanol solution (preparation of the first electolyde). After heat treatment at 500°C for 15 minutes, the solution in which 1g of TiO₂ was diluted in 10mL ethanol was coated with 1g of TiO₂ paste using a spin coating method. The thickness can be adjusted with a TiO₂ paste concentration and a spin speed. Subsequently, the heat treatment was conducted at a temperature of 500°C for 1 hour.

Each of the solutions prepared in Examples 1 to 7 and the Comparative Example was dropped on the FTO glass that TiO₂ films were coated (2.5 x 2.5 cm²) and then coated with a spin coating method. Toluene was dropped as a nonsolvent 10 seconds before completion of the coating. The heat treatment was conducted on a hot plate at a temperature of 100°C for 10 minutes. Then, 60 mM Spiro-OMeTAD/Li-TFSI/tert-butylpyridine/ chlorobenzene (Aldrich) was coated by a spin coating to produce a hole transport layer. The anode was scrapped with a size of width 2.5 cm and length 0.5 cm, placed on a mask and deposited with Au to produce an electrode (second electrode).

The efficiency of the solar cells was measured by using the solar cells thus prepared and the results are shown in Table 1 below and Fig. 1. Humidity at the time of measurement was maintained at 20%. Table 1 below shows the results of the first measurement, and Fig. 1 shows the power conversion efficiency measured wht the lapse of time for the solar cells.

**[Table 1]**

| | Short-circuit current density (mA/cm²) | Open-circuit voltage (V) | Figure of merit (%) | Power conversion efficiency (%) |
|---|---|---|---|---|
| Comparative Example | 19.42 | 1.079 | 0.73 | 15.30 |
| Example 1 | 20.77 | 1.075 | 0.71 | 15.88 |
| Example 2 | 19.97 | 1.100 | 0.73 | 16.32 |

As shown in Table 1 and Fig.1, it could be confirmed that in the case of the Comparative Example, the power conversion efficiency decreases rapidly with the lapse of time after the first measurement, whereas in the case of the Examples, the power conversion efficiency is maintained at a high number for a long period of time.

Accordingly, for the solar cell comprising the organic-inorganic hybrid perovskite compound according to the present invention, since deuterium is substituted, a zero point energy becomes low and the chemical stability of the perovskite compound increase, thereby increasing the stability of the solar cells.

## Claims

1. A solar cell comprising an organic-inorganic hybrid perovskite compound, wherein the organic-inorganic hybrid perovskite compound is represented by the following chemical formula 1 or 2:
[Formula 1] AMX₃
[Formula 2] A₂MX₄
in the above formulas,
A is CD₃₋ₐHₐN⁺D_{3-b}H_{b}, wherein a is a real number from 0 to 3, b is a real number from 0 to 3, except i) when a is 3 and b is 3, ii) when a is 3 and b is 0, or iii) when a is 0 and b is 3,
M is a divalent metal ion, and
X is a halogen ion.

2. The solar cell of claim 1, wherein a and b are 0 in the organic-inorganic hybrid perovskite compound.

3. The solar cell of claim 1, wherein M is Pb²⁺, Sn²⁺, Ti²⁺, Nb²⁺, Zr²⁺ or Ce²⁺ in the organic-inorganic hybrid perovskite compound.

4. The solar cell of claim 1, wherein X is Cl⁻, Br⁻ or I⁻ in the organic-inorganic hybrid perovskite compound.

5. The solar cell of any one of claims 1 to 4, wherein the solar cell has the following structure:
a first electrode including a conductive transparent substrate;
a light absorbing layer, formed on the first electrode, comprising the organic-inorganic hybrid perovskite compound defined in anyone of claims 1 to 4;
a second electrode disposed opposite to the first electrode in which the light-absorbing layer is formed; and
an electrolyte layer located between the first electrode and the second electrode.

6. A process of providing a solar cell, comprising preparing the organic-inorganic hybrid perovskite compounds represented by the chemical formula 1 or 2 defined in claim 1,
wherein the preparation of these organic-inorganic hybrid perovskite compounds comprises the following steps:
1) mixing a compound represented by the following chemical formula 3 with a compound represented by the following chemical formula 4, and
2) heat-treating the mixture:
[Formula 3] AX
[Formula 4] MX₂
in the above formulas, A, M and X are the same as defined in claim 1.

## Patentansprüche

1. Solarzelle, umfassend eine organisch-anorganische Hybrid-Perovskit-Verbindung, wobei die organisch-anorganische Hybrid-Perovskit-Verbindung durch die folgende chemische Formel 1 oder 2 dargestellt ist:
[Formel 1] AMX₃
[Formel 2] A₂MX₄
wobei in den obigen Formeln
A für CD₃₋ₐHₐN⁺D_{3-b}H_{b} steht, wobei a eine reelle Zahl von 0 bis 3 ist, b eine reelle Zahl von 0 bis 3 ist, ausgenommen i) wenn a gleich 3 ist und b gleich 3 ist, ii) wenn a gleich 3 ist und b gleich 0 ist, oder iii) wenn a gleich 0 ist und b gleich 3 ist,
M für ein zweiwertiges Metallion steht, und
X für ein Halogen-Ion steht.

2. Solarzelle gemäß Anspruch 1, wobei a und b in der organisch-anorganischen Hybrid-Perovskit-Verbindung gleich 0 sind.

3. Solarzelle gemäß Anspruch 1, wobei M in der organisch-anorganischen Hybrid-Perovskit-Verbindung für Pb²⁺, Sn²⁺, Ti ²⁺, Nb²⁺, Zr²⁺ oder Ce²⁺ steht.

4. Solarzelle gemäß Anspruch 1, wobei X in der organisch-anorganischen Hybrid-Perovskit-Verbindung für Cl⁻, Br⁻ oder I⁻ steht.

5. Solarzelle gemäß einem der Ansprüche 1 bis 4, wobei die Solarzelle den folgenden Aufbau aufweist:
eine erste Elektrode mit einem leitfähigen transparenten Substrat;
eine auf der ersten Elektrode ausgebildete lichtabsorbierende Schicht, welche die in einem der Ansprüche 1 bis 4 definierte organisch-anorganische Hybrid-Perovskit-Verbindung umfasst;
eine zweite Elektrode, die der ersten Elektrode, in der die lichtabsorbierende Schicht ausgebildet ist, gegenüberliegt; und
eine Elektrolytschicht, die sich zwischen der ersten Elektrode und der zweiten Elektrode befindet.

6. Verfahren zur Herstellung einer Solarzelle, umfassend die Herstellung der organisch-anorganischen Hybrid-Perovskit-Verbindungen der in Anspruch 1 definierten chemischen Formel 1 oder 2,
wobei die Herstellung dieser organisch-anorganischen Hybrid-Perovskit-Verbindungen die folgenden Schritte umfasst:
1) Mischen einer Verbindung der folgenden chemischen Formel 3 mit einer Verbindung der folgenden chemischen Formel 4, und
2) Wärmebehandeln der Mischung:
[Formel 3] AX
[Formel 4] MX₂
wobei in den obigen Formeln A, M und X genauso wie in Anspruch 1 definiert sind.

## Revendications

1. Cellule solaire comprenant un composé de pérovskite hybride organique-inorganique, dans laquelle le composé de pérovskite hybride organique-inorganique est représenté par la formule chimique 1 ou 2 suivante :
[Formule 1] AMX₃
[Formule 2] A₂MX₄
dans les formules ci-dessus,
A est CD₃₋ₐHₐN⁺D_{3-b}H_{b}, dans laquelle a est un nombre réel de 0 à 3, b est un nombre réel de 0 à 3, sauf i) lorsque a vaut 3 et b vaut 3, ii) lorsque a vaut 3 et b vaut 0, ou iii) lorsque a vaut 0 et b vaut 3,
M est un ion métallique divalent, et
X est un ion halogénure.

2. Cellule solaire selon la revendication 1, dans laquelle a et b valent 0 dans le composé de pérovskite hybride organique-inorganique.

3. Cellule solaire selon la revendication 1, dans laquelle M est Pb²⁺, Sn²⁺, Ti²⁺, Nb²⁺, Zr²⁺ ou Ce²⁺ dans le composé de pérovskite hybride organique-inorganique.

4. Cellule solaire selon la revendication 1, dans laquelle X est Cl⁻, Br⁻ ou I⁻ dans le composé de pérovskite hybride organique-inorganique.

5. Cellule solaire selon l'une quelconque des renvendications 1 à 4, dans laquelle la cellule solaire a la structure suivante :
une première électrode incluant un substrat transparent conducteur ;
une couche d'absorption de la lumière, formée sur la première électrode, comprenant le composé de pérovskite hybride organique-inorganique défini dans l'une quelconque des revendications 1 à 4 ;
une seconde électrode disposée de manière opposée à la première électrode dans laquelle la couche d'absorption de la lumière est formée ; et
une couche électrolytique située entre la première électrode et la seconde électrode.

6. Procédé de fourniture d'une cellule solaire, comprenant une préparation des composés de pérovskite hybride organique-inorganique représentés par la formule chimique 1 ou 2 définie dans la revendication 1,
dans lequel la préparation de ces composés de pérovskite hybride organique-inorganique comprend les étapes suivantes :
1) de mélange d'un composé représenté par la formule chimique 3 qui suit avec un composé représenté par la formule chimique 4 qui suit, et
2) de traitement thermique du mélange :
[Formule 3] AX
[Formule 4] MX₂
dans les formules ci-dessus, A, M et X sont identiques à ceux définis dans la revendication 1.
